# EUROPEAN PATENT APPLICATION

(11) **EP 4 538 384 A1**
(43) Date of publication of application: **16.04.2025**
(21) Application number: 23819912.9
(22) Date of filing: 09.06.2023
(51) Int. Cl.: C12Q 1/04, C12N 5/074, C12N 5/10, C12N 15/09, C12Q 1/686, C12Q 1/6876

(54) **DETECTION METHOD AND DETECTION REAGENT FOR UNDIFFERENTIATED PLURIPOTENT STEM CELLS**

(30) Priority: 10.06.2022 JP 2022094396
(71) Applicant: Kyoto University, Kyoto-shi, Kyoto 606-8501 (JP); Rege Nephro Co., Ltd., Kyoto-shi, Kyoto, 606-8501 (JP)
(72) Inventor: OSAFUNE, Kenji, Kyoto-shi, Kyoto 606-8501 (JP); TSUJIMOTO, Hiraku, Kyoto-shi, Kyoto 606-8501 (JP)
(74) Representative: Hoffmann Eitle
(86) International application number: PCT/JP2023/021545
(87) International publication number: WO 2023/238932

(57) **Abstract**

An object of the present invention is to provide a method and a reagent for detecting an undifferentiated pluripotent stem cell(s). The object is achieved by being able to detect a few residual undifferentiated pluripotent stem cells through measurement of the level of RNA expressed from the MIR302CHG gene.

## Description

### Technical Field

The present invention relates to a method for detecting an undifferentiated pluripotent stem cell(s) and a reagent for detecting the same.

### Background Art

The use of renal progenitor cells (nephron progenitor cells) obtained by differentiation induction from human pluripotent stem cells such as human iPS cells (iPSCs) and human ES cells for the treatment of renal diseases such as renal failure is under investigation. However, in clinical use, cell preparations made from iPS cells in clinical application have a number of problems of tumorigenicity caused by residual iPS cells.

Therefore, it is essential to confirm the absence of residual iPS cells in and guarantee the safety of cell preparations. However, it is very difficult to detect and evaluate a few residual iPS cells against a large amount of cell preparations. Therefore, it is important to perform detection through the identification of substances that are not present in nephron progenitor cells but are present in large amounts in iPS cells.

Methods for detecting residual iPS cells have been reported, such as detection by an iPS cell culture amplification method (Non Patent Literature 1 and Non Patent Literature 2) and detection of LIN28A by PCR (e.g., Non Patent Literature 3). However, culture conditions used for the iPS cell culture amplification method are problematic in that nephron progenitor cells are also highly efficiently cultured and amplified, making culture amplification of iPS cells alone impossible, and LIN28A is expressed to some extent in nephron progenitor cells, as well as in pancreatic and liver organoids into which iPS cells have differentiated. Hence, these methods are difficult to be employed to detect iPS cells. Therefore, a new method for detecting iPS cells, which can be used in nephron progenitor cells, pancreatic progenitor cells, hepatic progenitor cells, or the like, has been required.

### Citation List

### Patent Literature

Patent Literature 1: Japanese Patent Laid-Open No. 2013-158325

### Non Patent Literature

Non Patent Literature 1: Watanabe T. et al., Cryotherapy 2021; 23: 2: 176-183
Non Patent Literature 2: Tano K. et al., PLOS ONE 2014; 9: 10.
Non Patent Literature 3: Kuroda T. et al., Regenerative Therapy 2015; 2: 17-23.

### Summary of Invention

### Technical Problem

An object of the present invention is to provide a method for detecting an undifferentiated pluripotent stem cell(s), such as a residual iPS cell(s) that are present in a cell population containing a nephron progenitor cell(s), etc., which has(ve) been obtained by differentiation induction from an iPS cell(s), and a reagent therefor.

### Solution to Problem

The present inventors conducted extensive research to solve the above problems. As a result, the present inventors discovered that measuring the level of RNA expressed from a MIR302CHG gene can detect a few undifferentiated pluripotent stem cells remaining in a cell population and the like, such as those obtained by differentiation induction from a pluripotent stem cell(s), leading to the completion of the present invention.

The present invention provides the following [1] to [15].
[1] A method for detecting an undifferentiated pluripotent stem cell(s), including (A) a step of measuring the level of RNA expressed from MIR302CHG in a cell population that may contain an undifferentiated pluripotent stem cell(s).
[2] The method according to [1], wherein the pluripotent stem cell(s) is an induced pluripotent stem (iPS) cell(s).
[3] The method according to [1] or [2], wherein the RNA expressed from MIR302CHG is a splice variant ENST00000509938.1 and/or a splice variant ENST00000505215.1.
[4] The method according to [3], wherein the RNA expressed from MIR302CHG is the splice variant ENST00000509938.1.
[5] The method according to any one of [1] to [4], wherein
   the RNA expressed from MIR302CH is
   (a) a nucleotide sequence of SEQ ID NO: 2 or SEQ ID NO: 3, or
   (b) a nucleotide sequence having an identity of 90% or more with the nucleotide sequence of SEQ ID NO: 2 or SEQ ID NO: 3.
[6] The method according to any of [1] to [5], wherein the step (A) includes a step of determining amplifiability by quantitative PCR or a step of determining amplifiability by 2-step ddPCR (Droplet digital PCR).
[7] The method according to [6], wherein the step of determining amplifiability by quantitative PCR comprises using an oligonucleotide primer set(s) having one or more pairs of sequences selected from the group consisting of SEQ ID NO: 4 and SEQ ID NO: 5, SEQ ID NO: 6 and SEQ ID NO: 7, SEQ ID NO: 8 and SEQ ID NO: 9, SEQ ID NO: 10 and SEQ ID NO: 11, SEQ ID NO: 12 and SEQ ID NO: 13, SEQ ID NO: 14 and SEQ ID NO: 15, SEQ ID NO: 16 and SEQ ID NO: 17, SEQ ID NO: 18 and SEQ ID NO: 19, SEQ ID NO: 20 and SEQ ID NO: 21, SEQ ID NO: 22 and SEQ ID NO: 23, SEQ ID NO: 24 and SEQ ID NO: 25, SEQ ID NO: 26 and SEQ ID NO: 27, SEQ ID NO: 28 and SEQ ID NO: 29, SEQ ID NO: 30 and SEQ ID NO: 31, and SEQ ID NO: 32 and SEQ ID NO: 33.
[8] The method according to [6], wherein the step of determining amplifiability by 2-step ddPCR comprises using an oligonucleotide probe containing the nucleotide sequence of SEQ ID NO: 36 and an oligonucleotide primer set having the sequences of SEQ ID NO: 34 and SEQ ID NO: 35.
[9] The method according to any one of [1] to [8], including a step of determining that an undifferentiated pluripotent stem cell(s) contained in a cell population is detected when the level of RNA in the cell population measured in the step (A) is 0.000001 or more with respect to the level of RNA in the cell population consisting of a pluripotent stem cell(s) being set as 1.
[10] The method according to any of [1] to [9], including a step of enriching a fraction containing the undifferentiated pluripotent stem cell(s) by cell sorting using an undifferentiated pluripotent stem cell-specific antigen(s) prior to step (A).
[11] The method according to [10], wherein the undifferentiated pluripotent stem cell-specific antigen(s) is TRA-1-60.
[12] The method according to any of [1] to [11], wherein the cell population contains a nephron progenitor cell(s), a pancreatic progenitor cell(s), a hepatic progenitor cell(s), an ureteroblast(s), an interstitial progenitor cell(s), a vascular endothelial cell(s), a chondrocyte(s), a bile duct progenitor cell(s), an erythropoietin-producing cell(s), a neuron(s), a cardiomyocyte(s), and/or a pneumocyte(s) obtained by differentiation induction from a pluripotent stem cell(s), or a cell(s) obtained at a stage during differentiation from a pluripotent stem cell(s) into any of these cells.
[13] The method according to any one of [1] to [12], wherein the cell population contains a nephron progenitor cell(s) obtained by differentiation induction from a pluripotent stem cell(s) or a cell(s) obtained at a stage during differentiation from a pluripotent stem cell(s) into a nephron progenitor cell(s).
[14] A reagent kit for detecting an undifferentiated pluripotent stem cell(s), including (i) oligonucleotide primers for measuring the level of RNA expressed from MIR302CHG or (ii) a combination of the oligonucleotide primers and an oligonucleotide probe.
[15] The reagent kit according to [14], wherein (i) the oligonucleotide primers are an oligonucleotide primer set(s) having one or more pairs of sequences selected from the group consisting of SEQ ID NO: 4 and SEQ ID NO: 5, SEQ ID NO: 6 and SEQ ID NO: 7, SEQ ID NO: 8 and SEQ ID NO: 9, SEQ ID NO: 10 and SEQ ID NO: 11, SEQ ID NO: 12 and SEQ ID NO: 13, SEQ ID NO: 14 and SEQ ID NO: 15, SEQ ID NO: 16 and SEQ ID NO: 17, SEQ ID NO: 18 and SEQ ID NO: 19, SEQ ID NO: 20 and SEQ ID NO: 21, SEQ ID NO: 22 and SEQ ID NO: 23, SEQ ID NO: 24 and SEQ ID NO: 25, SEQ ID NO: 26 and SEQ ID NO: 27, SEQ ID NO: 28 and SEQ ID NO: 29, SEQ ID NO: 30 and SEQ ID NO: 31, and SEQ ID NO: 32 and SEQ ID NO: 33, or (ii) the combination of primers and a probe is the combination of the oligonucleotide primer set having the sequences of SEQ ID NO: 34 and SEQ ID NO: 35, and the oligonucleotide probe containing the nucleotide sequence of SEQ ID NO: 36.

### Advantageous Effects of Invention

According to the present invention, an undifferentiated pluripotent stem cell(s) remaining in a cell population containing a nephron progenitor cell(s) obtained by differentiation induction from a pluripotent stem cell(s) can be detected. This enables the evaluation of the safety of products for regenerative medicine and others (for cell therapy, transplant organ reconstruction, etc.) containing a human iPS cell-derived nephron progenitor cell(s).

It is also expected to detect a residual undifferentiated pluripotent stem cell(s) in other differentiated cell culture systems other than a nephron progenitor cell(s). Therefore, the present invention can also be applied to products for regenerative medicine and others using another differentiated cell(s) other than a human iPS cell-derived nephron progenitor cell(s), such as a pancreatic progenitor cell(s), a hepatic progenitor cell(s), an ureteroblast(s), an interstitial progenitor cell(s) (e.g., a renal interstitial progenitor cell(s)), a vascular endothelial cell(s), a chondrocyte(s), a bile duct progenitor cell(s), an erythropoietin-producing cell(s), a neuron(s), a cardiomyocyte(s), and a pneumocyte(s).

### Brief Description of Drawings

[FIG. 1] FIG. 1 depicts a volcano plot (left panel) of DEG and the list (right panel) of human iPSC marker candidates with log10 (FDR) > 5 and log2FC (fold change) < -10.
[FIG. 2] FIG. 2 is a graph showing TPM values of MIR302CHG, CUZD1, LIN28A and TBP in human iPSCs and Nephron Progenitor Cells (NPCs) with mean ± SEM (n=3).
[FIG. 3] FIG. 3 depicts a list of markers (log2FC <10 and FDR <10-5) extremely highly expressed in human iPSCs compared to day 4 cells (D4C) of NPC differentiation protocol.
[FIG. 4] FIG. 4 depicts graphs showing TPM values of three splice variants of MIR302CHG in human iPSCs and NPCs, variant ENST00000510655.1, variant ENST00000509938.1, and variant ENST00000505215.1 with mean ± SEM (n=3).
[FIG. 5] FIG. 5 depicts scatter plots showing TBP-normalized gene expression of MIR302CHG, CUZD1, and POU5F1 as measured by quantitative RT-PCR analysis on mixed cells of human iPSCs and NPCs (left panel) or mixed cells of human iPSCs and D4Cs (right panel). Dots and lines in the center of the scatter plots indicate the experimental data and the mean value of the data, respectively, and UD indicates samples for which the CT value was Undetermined.
[FIG. 6] FIG. 6 depicts a scatter plot of TBP-normalized dCT values as a result of quantitative RT-PCR of human iPSCs (iPSCs) and induced NPCs (NPCs) for 15 types of the MIR302CHG primer. UD indicates samples for which the CT value was Undetermined.
[FIG. 7] FIG. 7 depicts scatter plots showing the TBP-normalized gene expressions of MIR302CHG, CUZD1, and POU5F1 as measured by quantitative RT-PCR analysis on NPCs (left panel) or D4Cs (right panel), MACS negative fraction flow-through (NF), 10⁻⁴% iPSCs, MACS positive fraction flow-through (PF), and iPSCs. Dots and lines in the center of each scatter plot indicate the experimental data and the mean value, respectively.
[FIG. 8] FIG. 8 depicts a scatter plot of ddPCR estimated copy number ratios of TBP-normalized MIR302CHG as measured by Droplet digital PCR (ddPCR) using 15% of RT products.
[FIG. 9] FIG. 9 depicts scatter plots of TPM values of three splice variants of MIR302CHG in human iPSCs or human iPSC-derived pancreatic progenitor cells, a variant ENST00000510655.1, a variant ENST00000509938.1, and a variant ENST00000505215.1.
[FIG. 10] FIG. 10 depicts scatter plots of TPM values of three splice variants of MIR302CHG in human iPSCs or human iPSC-derived hepatocytes, the variant ENST00000510655.1, the variant ENST00000509938.1, and the variant ENST00000505215.1.
[FIG. 11] FIG. 11 depicts scatter plots of TPM values of three splice variants of MIR302CHG in human iPSCs or human iPSC-derived neural progenitor cells and neurons, the variant ENST00000510655.1, the variant ENST00000509938.1, and the variant ENST 00000505215.1.
[FIG. 12] FIG. 12 depicts scatter plots of TPM values of three splice variants of MIR302CHG in human iPSCs or human iPSC-derived cardiomyocytes, the variant ENST00000510655.1, the variant ENST00000509938.1, and the variant ENST00000505215.1.
[FIG. 13] FIG. 13 depicts scatter plots of TPM values of three splice variants of MIR302CHG in human ESCs or human ESC-derived lung progenitor cells or lung organoids, the variant ENST00000510655.1, the variant ENST00000509938.1, and the variant ENST 00000505215.1, and the variant ENST00000509938.1.
[FIG. 14] FIG. 14 depicts scatter plots of TPM values of three splice variants of MIR302CHG in human iPSCs, IVF-ESCs, nt-ESCs or human iPSCs, IVF-ESCs, nt-ESC-derived vascular endothelial cells, the variant ENST00000510655.1, the variant ENST00000509938. 1, and the variant ENST00000505215.1.
[FIG. 15] FIG. 15 depicts scatter plots of TPM values of three splice variants of MIR302CHG in human ESCs, anterior primitive streak, embryonic endoderm, anterior foregut, posterior foregut, and midgut/hindgut, the variant ENST00000510655.1, the variant ENST00000509938.1, and the variant ENST 00000505215.1.
[FIG. 16] FIG. 16 is a graph of MIR302CHG/TBP showing the results of analyzing the expression of MIR302CHG in renal interstitial progenitor cells (IPCs), ureteroblasts (ND) and human iPSCs by quantitative RT-PCR, followed by normalization by TBP (N=4 for IPC, N=5 for ND).

### Description of Embodiments

The present invention is described below.

The method for detecting an undifferentiated pluripotent stem cell(s) of the present invention includes the step of measuring the level of RNA expressed from MIR302CHG in a cell population that may contain an undifferentiated pluripotent stem cell(s).

### <An undifferentiated pluripotent stem cell(s)>

In the present invention, a pluripotent stem cell(s) is/are a stem cell(s) that is/are pluripotent and thus is/are capable of differentiating into many types of cells existing in the living body, and also has(ve) proliferative properties, including any type of cells that are induced to differentiate into a somatic cell(s) such as a nephron progenitor cell(s), a pancreatic progenitor cell(s), a hepatic progenitor cell(s), an ureteroblast(s), an interstitial progenitor cell(s) (e.g., a renal interstitial progenitor cell(s)), a vascular endothelial cell(s), a chondrocyte(s), a bile duct progenitor cell(s), an erythropoietin-producing cell(s), a neuron(s), a cardiomyocyte(s), and a pneumocyte(s).

Examples of pluripotent stem cells include, but are not particularly limited to, an embryonic stem (ES) cell(s), an induced pluripotent stem (iPS) cell(s), an embryonic stem (nt-ES) cell(s) derived from cloned embryos obtained by nuclear transfer, a sperm stem cell(s) ("GS cell(s)"), an embryonic germ cell(s) ("EG cell(s)"), and a pluripotent cell(s) (Muse cell(s)) derived from cultured fibroblasts or bone marrow stem cell(s). A preferred pluripotent stem cell(s) is/are an iPS cell(s) and an ES cell(s). A pluripotent stem cell(s) is/are preferably derived from a mammal(s) including a primate(s) and a rodent(s), more preferably from a primate(s), and even more preferably from a human(s).

Methods for producing an iPS cell(s) are known in the art, and an iPS cell(s) can be produced by, for example, introducing reprogramming factors into any type of a somatic cell(s). Examples of reprogramming factors include a gene(s) or a gene product(s) such as Oct3/4, Sox2, Sox1, Sox3, Sox15, Sox17, Klf4, Klf2, c-Myc, N-Myc, L-Myc, Nanog, Lin28, Fbx15, ERas, ECAT15-2, Tcl1, beta-catenin, Lin28b, Sall1, Sall4, Esrrb, Nr5a2, Tbx3Glis1, or miR302/367 cluster. These reprogramming factor(s) may be used alone or in combination. Examples of the combination of reprogramming factors include WO2007/069666, WO2008/118820, WO2009/007852, WO2009/032194, WO2009/058413, WO2009/057831, WO2009/075119, WO2009/079007, WO2009/091659, WO2009/101084, WO2009/101407, WO2009/102983, WO2009/114949, WO2009/117439, WO2009/126250, WO2009/126251, WO2009/126655, WO2009/157593, WO2010/009015, WO2010/033906, WO2010/033920, WO2010/042800, WO2010/050626, WO2010/056831, WO2010/068955, WO2010/098419, WO2010/102267, WO2010/111409, WO2010/111422, WO2010/115050, WO2010/124290, WO2010/147395, and WO2010/147612 described in Huangfu D, et al. (2008), Nat. Biotechnol., 26:795-797, Shi Y, et al. (2008), Cell Stem Cell, 2: 525-528, Eminli S, et al. (2008), Stem Cells 26:2467-2474, Huangfu D, et al. (2008), Nat. Biotechnol. 26: 1269-1275, Shi Y, et al. (2008), Cell Stem Cell, 3,568-574, Zhao Y, et al. (2008), Cell Stem Cell, 3: 475-479, Marson A, (2008), Cell Stem Cell, 3,132-135, Feng B, et al. (2009), Nat. Cell Biol. 11: 197-203, R. L. Judson et al., (2009), Nat. Biotechnol., 27:459-461, Lyssiotis CA, et al. (2009), Proc Natl Acad Sci USA. 106: 8912-8917, Kim JB. et al. (2009), Nature 461: 649-643, Ichida JK, et al. (2009), Cell Stem Cell 5: 491-503, Heng JC, et al. (2010), Cell Stem Cell 6: 167-74, Han J, et al. (2010), Nature 463: 1096-1100, Mali P, et al. (2010), Stem Cells 28: 713-720, Maekawa M, et al. (2011), Nature 474: 225-9, and Cell Stem Cell. 2011;8: 376-88. doi:10.1016/j.stem.2011.03.001.

Examples of somatic cells include, but are not limited to, a fetal (offspring) somatic cell(s), a neonatal (offspring) somatic cell(s), and a mature healthy or diseased somatic cell(s), and also include all of primary cultured cells, passaged cells, and cell lines. Specific examples of somatic cells include (1) a tissue stem cell(s) (a somatic stem cell(s)) such as a neural stem cell(s), a hematopoietic stem cell(s), a mesenchymal stem cell(s), and a dental pulp stem cell(s), (2) a tissue progenitor cell(s), (3) a differentiated cell(s) such as a blood cell(s) (a peripheral blood cell(s), a cord blood cell(s), etc.), a lymphocyte(s), an epithelial cell(s), an endothelial cell(s), a muscle cell(s), a fibroblast(s) (such as a skin cell(s)), a hair cell(s), a hepatocyte(s), a gastric mucosal cell(s), an intestinal cell(s), a splenocyte(s), a pancreatic cell(s) (a pancreatic exocrine cell(s), etc.), a brain cell(s), a pneumocyte(s), a renal cell(s), and an adipocyte(s).

The term "an undifferentiated pluripotent stem cell(s)" refers to a pluripotent stem cell(s) that has(ve) the characteristic(s) of a pluripotent stem cell(s), such as pluripotency and proliferative properties, and that has(ve) not been induced to differentiate or have not undergone differentiation. A pluripotent stem cell(s) that has(ve) not undergone differentiation may be, for example, a cell(s) that has(ve) been induced to differentiate into any type of cells but has(ve) not lost their pluripotency or pluripotency and proliferative potential. That is, the an undifferentiated pluripotent stem cell(s) may be a pluripotent stem cell(s) that remain(s) in a cell population that has not been induced to differentiate or has been induced to differentiate into any type of cells from a pluripotent stem cell(s).

Thus, the undifferentiated pluripotent stem cell(s) may be specifically an ES cell(s), an iPS cell(s), a nt-ES cell(s), a GS cell(s), an EG cell(s), a Muse cell(s), etc., and specifically an ES cell(s), an iPS cell(s), a nt-ES cell(s), a GS cell(s), an EG cell(s), a Muse cell(s), etc., remaining in a cell population obtained by differentiation induction from a pluripotent stem cell(s) into any type of cells.

### <Cell population>

In the present invention, a cell population that may contain an undifferentiated pluripotent stem cell(s) is not particularly limited, as long as the cell population may contain an undifferentiated pluripotent stem cell(s). However, the cell population is preferably obtained by inducing a pluripotent stem cell(s) to differentiate into a specific lineage, i.e., the cell population that is obtained by differentiation induction from a pluripotent stem cell(s).

It is known to be possible that an undifferentiated pluripotent stem cell(s) remain(s) in a cell population obtained by differentiation induction from a pluripotent stem cell(s). Therefore, there is a case in which an undifferentiated pluripotent stem cell(s) remain(s) in a cell population containing a cell(s) obtained by differentiation induction from a pluripotent stem cell(s). However, even if an undifferentiated pluripotent stem cell(s) do(es) not actually remain in a cell population, such a cell population shall be considered as a "cell population that may contain an undifferentiated pluripotent stem cell(s)" in the present invention.

Preferable examples of the cell population that may contain an undifferentiated pluripotent stem cell(s) include cell population which contains a nephron progenitor cell(s), a pancreatic progenitor cell(s), a hepatic progenitor cell(s), an ureteroblast(s), an interstitial progenitor cell(s) (for example, a renal interstitial progenitor cell(s)), a vascular endothelial cell(s), a chondrocyte(s), a bile duct progenitor cell(s), an erythropoietin-producing cell(s), a neuron(s), a cardiomyocyte(s), and/or a pneumocyte(s), and more preferable examples include cell population which contains a nephron progenitor cell(s), an ureteroblast(s) and/or an interstitial progenitor cell(s) (for example, a renal interstitial progenitor cell(s)), and further more preferable examples include cell population which contains a nephron progenitor cell(s).

Further, the cell population that may contain an undifferentiated pluripotent stem cell(s) is: preferably a cell population that contains a nephron progenitor cell(s), a pancreatic progenitor cell(s), a hepatic progenitor cell(s), an ureteroblasts, an interstitial progenitor cell(s) (for example, a renal interstitial progenitor cell(s)), a vascular endothelial cell(s), a chondrocyte(s), a bile duct progenitor cell(s), an erythropoietin-producing cell(s), a neuron(s), a cardiomyocyte(s), and/or a pneumocyte(s) that is/are obtained by inducing the differentiation of a pluripotent stem cell(s), or a cell(s) (for example, a mesoderm cell(s), an endoderm cell(s), a foregut cell(s) or a cell(s) at or later than these stages, etc.) that arise(s) from a pluripotent stem cell(s) at stages during differentiation into any of these cell(s); more preferably a cell population that contain a nephron progenitor cell(s), an ureteroblast(s), and/or an interstitial progenitor cell(s) (for example, a renal interstitial progenitor cell(s)) obtained by inducing the differentiation of pluripotent stem cell(s), or cell(s) obtained at stages during differentiation from a pluripotent stem cell(s) into a nephron progenitor cell(s), an ureteroblast(s), and/or an interstitial progenitor cell(s) (for example, a renal interstitial progenitor cell(s)); and even more preferably a cell population that contains a nephron progenitor cell(s) obtained by differentiation of a pluripotent stem cell(s), or a cell(s) obtained at stages during differentiation from a pluripotent stem cell(s) into a nephron progenitor cell(s).

Any known method can be employed to induce differentiation from a pluripotent stem cell(s) into a nephron progenitor cell(s), a pancreatic progenitor cell(s), a hepatic progenitor cell(s), an ureteroblasts, an interstitial progenitor cell(s) (e.g., a renal interstitial progenitor cell(s)), a vascular endothelial cell(s), a chondrocyte(s), a bile duct progenitor cell(s), an erythropoietin-producing cell(s), a neuron(s), a cardiomyocyte(s), and/or a pneumocyte(s). For example, differentiation may be induced by adding an appropriate differentiation inducing factor(s) to a culture medium.

Specific examples of such a method for inducing differentiation from a pluripotent stem cell(s) into a nephron progenitor cell(s) include methods described in Tsujimoto, H. et al. (2020), Cell Rep. 31, 107476, WO2020/213734, Morizane, R. et al. (2015) Nat. Biotechnol. 33, 1193-1200, and Li, Z. et al. (2016) Cell Stem Cell 19, 516-529, 2016.

A specific example of a method for inducing differentiation from a pluripotent stem cell(s) into a pancreatic progenitor cell(s) is a method described in Kimura, A.et al. (2020) Cell Chem. Biol. 27, 1561-1572.e7.

Specific examples of a method for inducing differentiation from a pluripotent stem cell(s) into a hepatic progenitor cell(s) include methods described in WO2016/104717, Kotaka, M. et al. (2017) Sci. Rep. 7, 1-13. and Ouchi, R. et al. (2019) Cell Metab. 1-11. doi: 10. 1016/j.cmet.2019.05.007.

Specific examples of a method for inducing differentiation from a pluripotent stem cell(s) into an ureteroblast(s) include a method described in Mae, SI, et al. Cell Rep, 32 (2020), 10.1016/j.celrep.2020.107963.

A specific example of a method for inducing differentiation from a pluripotent stem cell(s) into a vascular endothelial cell(s) and a chondrocyte(s) is a method described in Tsujimoto H, et al. Cell Rep. 2020 Apr 7;31(1):107476. doi: 10. 1016/j.celrep.2020.03.040.

Specific examples of a method for inducing differentiation from a pluripotent stem cell(s) into an interstitial progenitor cell(s) include methods described in WO/2023/017848, and Takasato M, et al., Nature. 2015;526: 564-568. doi:10.1038/nature15695.

A specific example of a method for inducing differentiation from a pluripotent stem cell(s) into a bile duct progenitor cell(s) is a method described in Matsui S, et al., Stem Cell Res. 2019 Mar; 35: 101400. doi: 10.1016/j. scr. 2019. 101400.

A specific example of a method for inducing differentiation from a pluripotent stem cell(s) into an erythropoietin-producing cell(s) is a method described in Katagiri N, et al. Sci Rep. 2021 Feb 16;11(1):3936. doi: 10.1038/s41598-021-83431-6.

A specific example of a method for inducing differentiation from a pluripotent stem cell(s) into a neuron(s) is a method described in Chen J, et al. PLoS One. 2013; 8: e75682. doi: 10.1371/journal.pone.0075682.

A specific example of a method for inducing differentiation from a pluripotent stem cell(s) into a cardiomyocyte(s) is a method described in Banovich NE, et al. Genome Res. 2018;28: 122-131. doi:10.1101/gr.224436.117.

A specific example of a method for inducing differentiation from a pluripotent stem cell(s) into a pneumocyte(s) is a method described in Jacob A, et al., Cell Stem Cell. 2017; 21: 472-488.e10. doi: 10.1016/j.stem.2017.08.014 or Kerschner LJ, et al., J Cell Mol Med. 2020; 24: 9853-9870. doi: 10.1111/jcmm.15568.

### <MIR302CHG>

MIR302CHG is a known gene not coding for proteins, which contains a cluster encoding miRNA, miR-302/367, at its locus.

The RNA expressed from MIR302CHG is known to contain three long-chain non-coding RNA (lncRNA) variants, ENST00000510655.1 (variant 655), ENST00000509938.1 (variant 938), and ENST00000505215.1 (variant 215).

Specifically, examples of the RNA expressed from MIR302CHG include the above miRNAs and their precursors, as well as the above lncRNAs.

The RNA expressed from MIR302CHG to be measured in the present invention is preferably lncRNA. In addition, of the above three lncRNA variants expressed from MIR302CHG, one or more thereof are preferably arbitrary variants, and more preferably variant 938 and/or variant 215, and even more preferably variant 938.

Specific examples of nucleotide sequences prepared by deletion of polyA sequence from each of the nucleotide sequences of variant 655, variant 938 and variant 215 are shown in SEQ ID NO: 1, SEQ ID NO: 2 and SEQ ID NO: 3. Thus, RNA to be measured in the method of the present invention may be RNA having a nucleotide sequence that contains the sequence of SEQ ID NO: 1, SEQ ID NO: 2 or SEQ ID NO: 3, or a sequence substantially identical to the sequence of SEQ ID NO: 1, SEQ ID NO: 2 or SEQ ID NO: 3. More suitably, RNA to be measured in the method of the present invention is RNA having a nucleotide sequence that contains the sequence of SEQ ID NO: 2, or a sequence substantially identical to the sequence of SEQ ID NO: 2. Note that a base may be modified in the cell(s), such as 5'-methylated cytosine. RNA having a sequence that contains such a modified base in the sequence of SEQ ID NO: 1, SEQ ID NO: 2 or SEQ ID NO: 3 or a sequence that is substantially identical to the sequence of SEQ ID NO: 1, SEQ ID NO: 2 or SEQ ID NO: 3 is also subjected to the measurement of the present invention. In the method of the present invention, the complementary RNA or complementary DNA of the RNA having the sequence of SEQ ID NO: 1, SEQ ID NO: 2 or SEQ ID NO: 3 or a sequence substantially identical to the sequence of SEQ ID NO: 1, SEQ ID NO: 2 or SEQ ID NO: 3 may also be measured.

In the present invention, the term "substantially identical sequence" refers to a nucleotide sequence having an identity of 80% or more, preferably 90% or more, more preferably 95% or more, even more preferably 97% or more, and particularly preferably 99% or more with respect to the entire nucleotide sequence exemplified above, or a nucleotide sequence in which one or several nucleotides at one or several positions have been substituted, deleted, modified, or inserted.

The above "one or several" specifically means preferably 1 to 20, more preferably 1 to 10, further preferably 1 to 5, and particularly preferably 1 to 3.

### <Measurement of RNA level>

In the present invention, "measuring the level of RNA or RNA level" means measuring the amount of RNA, and even if the measured amount is below the detection limit, this is included in "measuring the level of RNA".

The method for measuring the level of RNA is not particularly limited as long as it is a method that can measure the amount of RNA contained in a cell population, and any known method can be used. Examples of such methods include a measurement method that utilizes probe hybridization, a measurement method that utilizes nucleic acid amplification, a measurement method that utilizes mass spectrometry, and a measurement method that utilizes sequencing. Examples of the measurement method using nucleic acid amplification include a method for determining amplifiability by quantitative RT-PCR, which combines Reverse Transcription (RT) reaction and PCR, a method for determining amplifiability by 2-step (RT) ddPCR, which combines Reverse Transcription (RT) reaction and 2-step ddPCR, and a one-step ddPCR that directly determines the amplifiability of RNA. Examples of the method for measuring the level of RNA of the present invention preferably include a method for determining amplifiability by quantitative RT-PCR or a method for determining amplifiability by 2-step (RT) ddPCR.

Specifically, for example, the measurement of the level of RNA is preferably performed by extracting RNA from a cell population, synthesizing DNA from the extracted RNA via reverse transcription reaction, and thus determining amplifiability by quantitative PCR or by 2-step ddPCR.

The method for extracting RNA from a cell population is not particularly limited as long as it is a method that can extract RNA expressed from MIR302CHG, and can be appropriately selected from known methods. Specific examples thereof include the AGPC method. The extracted RNA may be total RNA, or a portion of RNA that contains RNA expressed from MIR302CHG.

Furthermore, the RNA extracted from a cell population is preferably subjected to a treatment to remove DNA. Such a treatment for removing DNA can be appropriately selected from known treatment methods, and an example thereof is a treatment method that involves adding a DNA degrading enzyme (DNase).

The step of synthesizing DNA from extracted RNA by reverse transcription reaction is typically carried out using reverse transcriptase. The method for synthesizing DNA by reverse transcription reaction can be appropriately selected from known methods, but DNA is preferably synthesized using a primer targeting a polyA sequence or a random sequence, the extracted RNA as a template and reverse transcriptase, and is more preferably synthesized using a primer targeting a polyA sequence, the extracted RNA as a template, and reverse transcriptase. The reverse transcription reaction may be carried out simultaneously with quantitative PCR or 2-step ddPCR described below.

The method for determining amplifiability by quantitative PCR can be appropriately selected from known methods, such as the SYBR (trademark) Green method and the TaqMan (trademark) method. Performing the aforementioned step of synthesizing DNA from RNA by reverse transcription reaction and quantitative PCR successively or simultaneously is also referred to as quantitative RT-PCR.

It is preferable to determine at least the amplifiability of RNA expressed from MIR302CHG by quantitative PCR. Examples of oligonucleotide primers for amplifying RNA expressed from MIR302CHG, or a portion of its DNA complementary sequence or RNA complementary sequence in quantitative PCR include combinations of SEQ ID NO: 4 and SEQ ID NO: 5, SEQ ID NO: 6 and SEQ ID NO: 7, SEQ ID NO: 8 and SEQ ID NO: 9, SEQ ID NO: 10 and SEQ ID NO: 1 1, SEQ ID NO: 12 and SEQ ID NO: 13, SEQ ID NO: 14 and SEQ ID NO: 15, SEQ ID NO: 16 and SEQ ID NO: 17, SEQ ID NO: 18 and SEQ ID NO: 19, SEQ ID NO: 20 and SEQ ID NO: 21, SEQ ID NO: 22 and SEQ ID NO: 23, SEQ ID NO: 24 and SEQ ID NO: 25, SEQ ID NO: 26 and SEQ ID NO: 27, SEQ ID NO: 28 and SEQ ID NO: 29, SEQ ID NO: 30 and SEQ ID NO: 31, and SEQ ID NO: 32 and SEQ ID NO: 33.

Thus, the amplifiability of RNA can be determined by quantitative PCR using preferably an oligonucleotide primer set(s) having one or more pairs of sequences selected from the group consisting of SEQ ID NO: 4 and SEQ ID NO: 5, SEQ ID NO: 6 and SEQ ID NO: 7, SEQ ID NO: 8 and SEQ ID NO: 9, SEQ ID NO: 10 and SEQ ID NO: 11, SEQ ID NO: 12 and SEQ ID NO: 13, SEQ ID NO: 14 and SEQ ID NO: 15, SEQ ID NO: 16 and SEQ ID NO: 17, SEQ ID NO: 18 and SEQ ID NO: 19, SEQ ID NO: 20 and SEQ ID NO: 21, SEQ ID NO: 22 and SEQ ID NO: 23, SEQ ID NO: 24 and SEQ ID NO: 25, SEQ ID NO: 26 and SEQ ID NO: 27, SEQ ID NO: 28 and SEQ ID NO: 29, SEQ ID NO: 30 and SEQ ID NO: 31, and SEQ ID NO: 32 and SEQ ID NO: 33.

The method for determining amplifiability by 2-step ddPCR is performed by ddPCR using a combination of oligonucleotide primers and an oligonucleotide probe. It is preferable to determine the amplifiability of RNA expressed from MIR302CHG by 2-step ddPCR. Examples of oligonucleotide primers and an oligonucleotide probe for amplifying RNA expressed from MIR302CHG or a portion of its complementary DNA sequence or complementary RNA sequence in 2-step ddPCR are an oligonucleotide primer set having the sequences of SEQ ID NO: 34 and SEQ ID NO: 35, and an oligonucleotide probe having the sequence of SEQ ID NO: 36.

Therefore, the amplifiability of RNA is determined by 2-step ddPCR using preferably DNA containing the nucleotide sequence of SEQ ID NO: 36 as an oligonucleotide probe and DNA having the sequences of SEQ ID NO: 34 and SEQ ID NO: 35 as an oligonucleotide primer set.

The level of RNA can be measured based on amplification by quantitative PCR or 2-step ddPCR, for example.

The level of RNA expressed from MIR302CHG may be evaluated by the absolute value (copy number) and may be determined that an undifferentiated pluripotent stem cell(s) is/are present when more than a certain level thereof is detected, e.g., 1 copy or more or 10 copies or more are detected.

The level of RNA expressed from MIR302CHG may be normalized by the expression level of a housekeeping gene (e.g., TATA-box binding protein (TBP) gene), and then evaluated with its value (relative amount). When the value is at a certain level or higher, the presence of an undifferentiated pluripotent stem cell(s) may be determined.

On the other hand, the level of RNA measured may be expressed as a value relative to the positive control. Specifically, for example, the measured level of RNA expressed from MIR302CHG in a cell population consisting of a pluripotent stem cell(s) is set to 1, and the measured RNA level may be expressed as a value relative thereto.

For example, the measured level of RNA expressed from MIR302CHG in a cell population consisting of a fixed number of a pluripotent stem cell(s) (first cell population) is set to 1, and the measured level of RNA expressed from MIR302CHG in a cell population that may contain an undifferentiated pluripotent cell(s) (second cell population), the number of which is the same as that of the first cell population, is expressed as a relative value, and then the presence or absence of an undifferentiated pluripotent stem cell(s) can be determined based on the value.

For example, it can be determined that an undifferentiated pluripotent stem cell(s) is/are present when the relative value is measured to be equal to or greater than 0.000001, 0.00001, 0.0001, or 0.001.

A cell population to be used in the method of the present invention may be purified, enriched or fractionated to any fraction prior to the step of measuring the level of RNA. Specifically, for example, a cell population that may contain an undifferentiated pluripotent stem cell(s) may be subjected to purification, enrichment, culture amplification, etc., using an antigen(s) specific to an undifferentiated pluripotent stem cell(s) as an indicator, or an mRNA switch targeting a nucleotide specific to an undifferentiated pluripotent stem cell(s), or culture conditions suitable for proliferation and adhesion of an undifferentiated pluripotent stem cell(s), etc., so as to enrich and/or fractionate into fractions that may contain an undifferentiated pluripotent stem cell(s). Examples of such an undifferentiated pluripotent stem cell-specific antigen(s) include(s) a protein(s), a glycopeptide(s), a glycoprotein(s), a glycolipid(s), and a glycan(s) specific to an undifferentiated pluripotent stem cell(s). A cell surface antigen(s) specific to an undifferentiated pluripotent stem cell(s) is/are preferred, and a specific example thereof is TRA-1-60. Examples of mRNA switches targeting an undifferentiated pluripotent stem cell-specific nucleotide(s) include miR-302a response switch (Fujita Y et al. Science Advance 2022 Jan 7;8(1):eabj 1793) and the like. Examples of conditions for undifferentiated pluripotent stem cell-specific proliferation and adhesion include Essential-8/LN521 culture amplification method (Tano et al., PLoS One. 2014 Oct 27;9(10)e110496). Alternatively, cell sorting is performed using a cell surface antigen(s) of a nephron progenitor cell(s), a pancreatic progenitor cell(s), a hepatic progenitor cell(s), anureteroblast(s), an interstitial progenitor cell(s) (e.g., a renal interstitial progenitor cell(s)), a vascular endothelial cell(s), a chondrocyte(s), a bile duct progenitor cell(s), an erythropoietin-producing cell(s), a neuron(s), a cardiomyocyte(s), a pneumocyte(s), etc. as an indicator(s), so as to remove these cell(s) from the cell population, and thus an undifferentiated pluripotent stem cell(s) may be enriched. Any known method of cell sorting can be selected, and an example thereof is a method using magnetic bead-based enrichment. The magnetic bead-based enrichment can be performed by MACS, for example.

### <Safety Assessment>

The use of the method of the present invention allows detection of an undifferentiated pluripotent stem cell(s) in products in which cell populations that may contain an undifferentiated pluripotent stem cell(s) are used, such as products for regenerative medicine and others produced using a nephron progenitor cell(s) derived from a human iPS cell(s), thus making it possible to assess safety.

Cell populations determined to be free of undifferentiated pluripotent stem cells by the method of the present invention may be further evaluated for tumorigenesis *in vivo.*

### <Kit for detecting an undifferentiated pluripotent stem cell(s)>

The present invention also relates to a reagent kit for detecting an undifferentiated pluripotent stem cell(s), including reagents for measuring the level of RNA expressed from MIR302CHG.

Specifically, the reagent kit of the present invention contains (i) oligonucleotide primers for measuring RNA expressed from MIR302CHG or (ii) a combination of the oligonucleotide primers and an oligonucleotide probe for measuring RNA expressed from MIR302CHG.

The oligonucleotide primers in (i) above are preferably an oligonucleotide primer set(s) having one or more pairs of sequences selected from the group consisting of, for example, SEQ ID NO: 4 and SEQ ID NO: 5, SEQ ID NO: 6 and SEQ ID NO: 7, SEQ ID NO: 8 and SEQ ID NO: 9, SEQ ID NO: 10 and SEQ ID NO: 11, SEQ ID NO: 12 and SEQ ID NO: 13, SEQ ID NO: 14 and SEQ ID NO: 15, SEQ ID NO: 16 and SEQ ID NO: 17, SEQ ID NO: 18 and SEQ ID NO: 19, SEQ ID NO: 20 and SEQ ID NO: 21, SEQ ID NO: 22 and SEQ ID NO: 23, SEQ ID NO: 24 and SEQ ID NO: 25, SEQ ID NO: 26 and SEQ ID NO: 27, SEQ ID NO: 28 and SEQ ID NO: 29, SEQ ID NO: 30 and SEQ ID NO: 31, and SEQ ID NO: 32 and SEQ ID NO: 33. As described above, such a oligonucleotide primer set can be used to determine the amplifiability of RNA by quantitative PCR. Thus, in addition, the reagent kit of the present invention may contain a reagent for quantitative PCR.

The combination of oligonucleotide primers and an oligonucleotide probe in (ii) above may be, for example, a combination of an oligonucleotide primer set having the sequences of SEQ ID NO: 34 and SEQ ID NO: 35 and an oligonucleotide probe having the sequence of SEQ ID NO: 36. As described above, such a combination can be used to determine the amplifiability of RNA by 2-step ddPCR. Thus, further the reagent kit of the present invention may contain a reagent for 2-step ddPCR or One step ddPCR.

### Examples

The present invention is described more specifically below with examples, but the present invention is not limited to the following embodiments.

### Pluripotent stem cells

Human iPS cells (human iPSCs) were used as pluripotent stem cells. Human iPSCs were maintained with feeder-free cultures using Stem Fit AK02N medium (Ajinomoto) or Stem Fit AK03N medium (Ajinomoto) on iMatrix-coated cell culture plates. The human iPSCs were passaged every 4 or 5 days using 0.5 mM EDTA/PBS (Thermo Fisher Scientific) and routinely tested for mycoplasma contamination.

### Nephron progenitor cells

Nephron progenitor cells (NPCs) were induced from human iPSCs by a method described in Tsujimoto, H. et al. (2020), Cell Rep. 31, 107476. Cells on day 4 after the initiation of differentiation from human iPSCs by the method described above were also used for analysis as Day 4 cells (D4C).

### Renal interstitial progenitor cells

Renal interstitial progenitor cells (IPCs) were obtained by differentiation induction from human iPSCs by a method described in WO/2023/017848, specifically by the following technique.

Human iPSCs were first enzyme-treated with a 1:1 mixture of TrypLE Select Enzyme (Thermo Fisher Scientific) and 0.5 mM EDTA/PBS for 5 minutes and then washed with PBS(-). Cells were then removed using a cell scraper, and then dissociated into single cells by gentle pipetting. On a day before initiation of differentiation, cells were seeded with 0.5 mL of Stem Fit AK02N (Ajinomoto Healthy Supply) supplemented with 10 µM Y-27632 and 2.4 µL/mL iMatrix-511 (Matrixome) into 24-well plates (Corning) at a density of 1.3 × 10⁴ cells/cm². After 24 hours (day 0), to a serum-free differentiation medium (hereinafter referred to as "basal medium") consisting of DMEM/F12 Glutamax (Thermo Fisher Scientific), B27 supplement minus vitamin A (Thermo Fisher Scientific), and 0.5 × Penicillin/Streptomycin, 5 µM CHIR99021 (Wako), 10 nM RA (Sigma), 1 ng/mL BMP4 (Peprotech), and 100 ng/mL bFGF (Wako) were added to initiate culturing. After another 24 hours (day 1), cells were cultured in a basal medium containing 5 µM CHIR99021, 100 ng/mL bFGF and 1 ng/mL BMP7 (R&D Systems). On day 2, the medium was switched to a basal medium containing 5 µM CHIR99021, 100 nM LDN193189 and 10 µM A83-01 (Wako). On day 3, the medium was switched to a basal medium containing 5 µM CHIR99021, 30 ng/mL bFGF, 10 ng/mL activin A (R&D Systems) and 30 µM Y-27632 (Wako). On day 4, cells were washed with PBS (-), treated with Accumax (innovative Cell Technologies), dissociated into single cells by gentle pipetting, and then seeded (3D culture) at a density of 2.0 × 10⁴ cells/well into 96-well low cell attachment plates (Thermo Fisher Scientific). The cells were cultured for 48 hours using a basal medium containing 5 µM CHIR99021, 30 ng/mL bFGF, 10 ng/mL activin A, and 30 µM Y-27632. Cells were then treated with a combination of 1 µM CHIR99021, 10 µM SB431542, 100 nM RA, 10 ng/mL IL-1b, and 500 nM Smoothsund (SMO) agonist (SAG) for 48 hours, further treated with a combination of 1 µM CHIR99021, 10 µM SB431542, 100 nM RA, and 500 nM Smoothsund (SMO) agonist (SAG) for 72 hours, and then cultured.

### Ureteroblasts

Ureteroblasts (ND) were induced from human iPSCs by a method described in Mae, SI, et al. Cell Rep, 32 (2020), 10.1016/j.celrep.2020.107963.

### RNA Sequencing (RNA-Seq) Analysis

Total RNA was isolated using the RNeasy Mini kit (QIAGEN) according to the manufacturer's recommended protocols. Next, sequence libraries were prepared using TruSeq Stranded Total RNA (Illumina). This library was sequenced using the NovaSeq SP Reagent Kit v1.5 (200 cycles) (Illumina) with 101-8-8-101 cycles. Gene expression was quantified using the analysis pipeline (2.3.4) used in the ENCODE project (https://www.encodeproject.org/pipelines/ENCPL002LPE/). GRCh38 in ENSEMBL 104 was used for the reference sequence. GRCh38 of GENCODE 38 was employed to define the genes. Comparative analysis of human iPSCs and NPCs was performed using two-group analysis (DESeq2) with the expected count calculated by the aforementioned pipeline (STAR-RSEM).

RNA-Seq data that have already been reported and published in a repository were analyzed using the following procedures. For NPCs and human iPSCs (201B7 cell line), transcript quantification was performed using Salmon with Homo sapiens GRCh38 cDNA gene annotation (Ensembl). The output from Salmon was processed using the R/Bioconductor package tximport to obtain gene expression values. DEG analysis was performed using iDEP (version 0.92). Enrichment analysis for the k-means-clustered top 1,000 DEGs was performed using iDEP through the biological process of Gene Ontology (GO).

From the RNA-seq data of pancreatic progenitor cells and liver organoids, gene-level normalized counts per million (CPM) or transcript-level transcripts per million (TPM) were obtained using GREIN.

### Quantitative RT-PCR

Quantitative RT-PCR was performed using the following procedure.

Total RNA was isolated using an RNeasy kit (QIAGEN) according to the manufacturer's recommended protocols. cDNA was synthesized from total RNA by performing a reverse transcription reaction according to standard protocols using ReverTra Ace (Toyobo) and oligo dT. Quantitative PCR was performed using QuantStudio 3 real-time PCR system (Thermo Fisher Scientific) and TB Green Premix Ex Taq II (Tli RNaseH Plus) (Takara Bio). Denaturation was performed at 95°C for 30 seconds, followed by 45 cycles of 95°C for 5 seconds and 60°C for 34 seconds. For data analysis of gene expression levels, quantification was performed according to the comparative Ct method, and the values were normalized to those of TBP, the housekeeping gene. PCR was performed at least three times for each sample. Table 1 lists the primers used for PCR.

**[Table 1]**

| Target gene | | Forward primer | Reverse primer |
|---|---|---|---|
| MIR302CHG (variant 938 and variant 215) | #1 | SEQ ID NO: 4 | SEQ ID NO: 5 |
| | #2 | SEQ ID NO: 6 | SEQ ID NO: 7 |
| | #3 | SEQ ID NO: 8 | SEQ ID NO: 9 |
| | #4 | SEQ ID NO: 10 | SEQ ID NO: 11 |
| | #5 | SEQ ID NO: 12 | SEQ ID NO: 13 |
| | #6 | SEQ ID NO: 14 | SEQ ID NO: 15 |
| | #7 | SEQ ID NO: 16 | SEQ ID NO: 17 |
| | #8 | SEQ ID NO: 18 | SEQ ID NO: 19 |
| | #9 | SEQ ID NO: 20 | SEQ ID NO: 21 |
| MIR302CHG (variant 938) | #10 | SEQ ID NO: 22 | SEQ ID NO: 23 |
| | #11 | SEQ ID NO: 24 | SEQ ID NO: 25 |
| | #12 | SEQ ID NO: 26 | SEQ ID NO: 27 |
| | #13 | SEQ ID NO: 28 | SEQ ID NO: 29 |
| | #14 | SEQ ID NO: 30 | SEQ ID NO: 31 |
| | #15 | SEQ ID NO: 32 | SEQ ID NO: 33 |
| POU5F1 | | SEQ ID NO: 37 | SEQ ID NO: 38 |
| CUZD1 | | SEQ ID NO: 39 | SEQ ID NO: 40 |
| TBP | | SEQ ID NO: 41 | SEQ ID NO: 42 |

### 2-Step ddPCR

2-step ddPCR was performed using the following procedure.

Total RNA was isolated according to the manufacturer's recommended protocols using an RNeasy kit (QIAGEN). cDNA was synthesized from total RNA by performing a reverse transcription reaction according to standard protocols using ReverTra Ace (Toyobo) and oligo dT.

cDNA obtained from total RNA was added to perform ddPCR using ddPCR Supermix for Probes (No dUTP) (Bio-Rad), 900 nM forward and reverse primers, and 250 nM probes labeled with FAM or HEX dye and ZEN-Iowa Black fluorescence quencher. First, droplets were generated in an 8-well cartridge by a QX200 Droplet Generator (Bio-Rad) or an Automated Droplet Generator (Bio-Rad) according to the manufacturer's instructions. This water-in-oil emulsions were transferred to 96-well plates, and PCR was performed using a C1000 Touch thermal cycler (Bio-Rad). Reaction conditions are as follows: 10 minutes of enzyme activation at 95°C and 40 cycles of a thermal profile comprising 30 seconds of denaturation at 94°C and 60 seconds of annealing/extension at 57.1°C. After PCR amplification, the products were denatured at 98°C for 10 minutes and then cooled at 4°C until fluorescence intensity was measured. The fluorescence intensity of each droplet obtained from the samples was measured using a QX200 Droplet Reader (Bio Rad). Positive droplets containing amplification products were distinguished from negative droplets and counted by applying a fluorescence amplitude threshold using QuantaSoft software. The threshold was determined manually based on the droplet distribution and was set at 4,000 for TBP and 7,000 for MIR302CHG. QuantaSoft software provides the concentration results in copies of target per microliter (copies/µL). The ratio of the copy number of target genes per copy number of TBP was calculated.

### Magnetic bead-based cell enrichment

Magnetic bead-based cell enrichment was performed using MACS (Miltenyi Biotec). Human iPSCs and NPCs were dissociated with Accumax (Innovative Cell Technologies, Inc.), and each cell suspension was centrifuged at 200 g for 7 minutes at 4°C. After centrifugation, the supernatant was discarded and each cell pellet was resuspended in MACS Rinsing solution (Miltenyi Biotec) supplemented with 0.5% BSA (Wako or Miltenyi Biotec) and 10 µM Y-27632. Cell suspensions containing human iPSCs and NPCs at a ratio of 1: 10⁶ were prepared, and then incubated with 1 µL of anti-TRA-1-60 microbeads, human (Miltenyi Biotec) per 10⁶ cells for 15 min at 4°C. Tapping was performed every five minutes. Next, the suspensions were then filtered using a 100-µm pore cell strainer (Falcon) and applied to an LS column with a MidiMACS separator (Miltenyi Biotec) attached thereto. The column was then washed twice using MACS Rinsing solution supplemented with 0.5% BSA. The labeled cells were applied and washed by gently pressing a syringe so that the drops fell at a rate of about 1 drop per second. The positive fraction was flushed out and collected by gently pressing the syringe at a rate of 2 to 3 drops per second. After centrifugation at 200 g for 5 minutes, cells obtained from the positive and negative fractions were respectively lysed using an RLT buffer (QIAGEN) supplemented with β-mercaptoethanol and stored at -80°C until analysis

### (Example 1)

### Detection of pluripotent stem cells

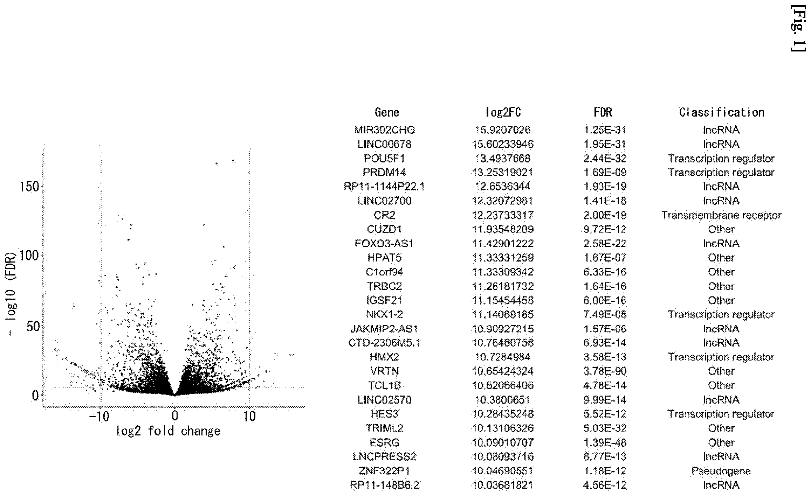

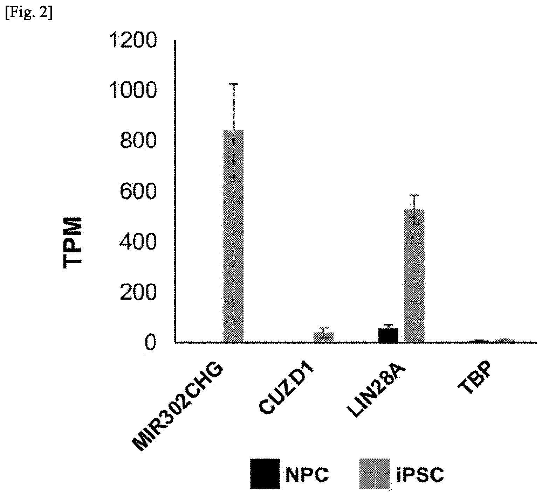

To identify genes that are highly expressed in human iPSCs but not in NPCs, RNA-seq analysis was performed including non-coding RNA and thus 26 extremely highly expressed genes were identified (log2FC < - 10 & FDR <10 ⁻⁵) (Fig. 1). Among these, MIR302CHG, which is related to lncRNA, was found to exhibit the value of each transcript (transcripts per million: TPM value) per million total transcripts, which was higher than that of LIN28A, which is used for conventional detection of residual iPS cells, and CUZD1, which is found to be differentially highly expressed in human iPSCs (data omitted) also in the analysis of deposited RNA-seq data (Fig. 2).

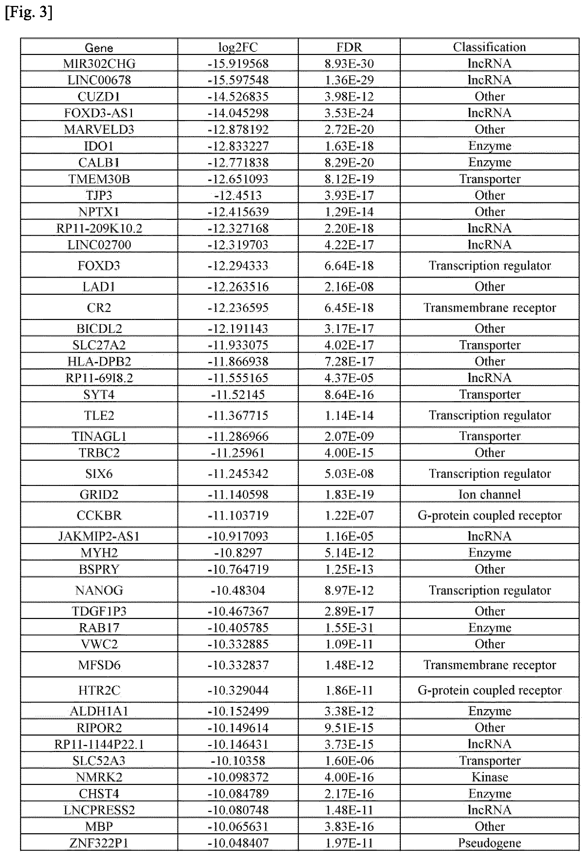

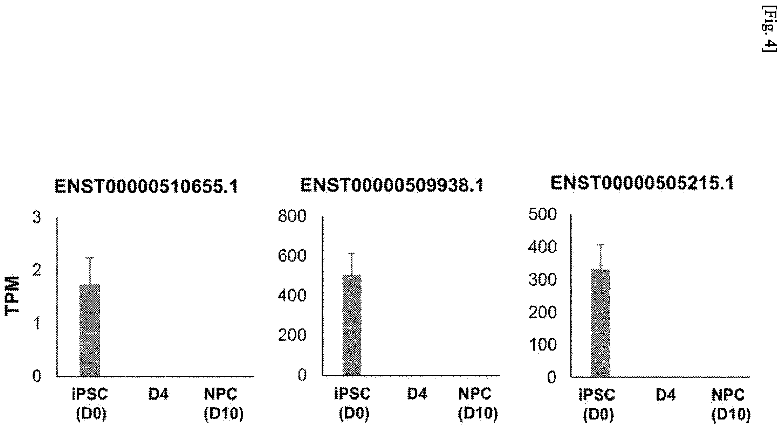

Furthermore, MIR302CHG was found to exhibit highly differential high expression thereof in iPSCs compared to not only that in NPCs after differentiation, but also that in cells on day 4 (D4C) of the NPC differentiation protocols (Fig. 3). Furthermore, all three splicing variants of MIR302CHG, ENST00000510655.1 (variant 655), ENST00000509938.1 (variant 938), and ENST00000505215.1 (variant 215), exhibited highly differential high expression in the above iPSCs. (Fig. 4).

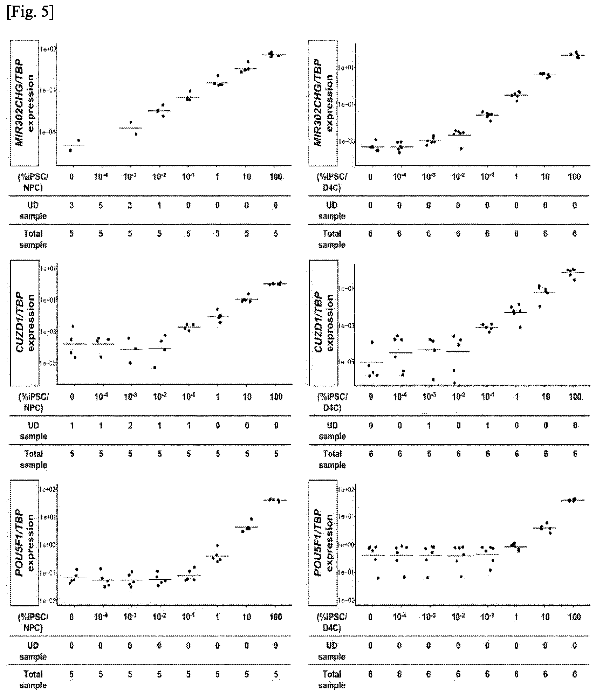

Since lncRNA expressed from MIR302CHG has a poly(A) 3' tail, reverse transcription (RT) reaction using oligo dT can be performed. Therefore, MIR302CHG, CUZD1, and pluripotency marker POU5F1 were analyzed by quantitative RT-PCR analysis using the mixtures of cDNAs derived from human iPSCs and NPCs or cells on day 4 of differentiation (D4C), which had been prepared by mixing them at 100% to 10⁻⁵ % in increments of 10⁻¹. For MIR302CHG, primers of the combination of SEQ ID NOS: 6 and 7 (corresponding to #2 in Table 1 above) were used. Fig. 5 depicts the results. The TBP-normalized MIR302CHG expression pattern was better at detecting iPSCs than the CUZD1 and POU5F1 expression patterns when the proportion of iPSCs was low. It was suggested that through analysis of the expression of MIR302CHG by quantitative RT-PCR, iPSC contamination of approximately 10⁻³ % (1 cell in 10⁵ cells) in NPC and 10⁻¹ (1 cell in 10³ cells) in D4C can be detected.

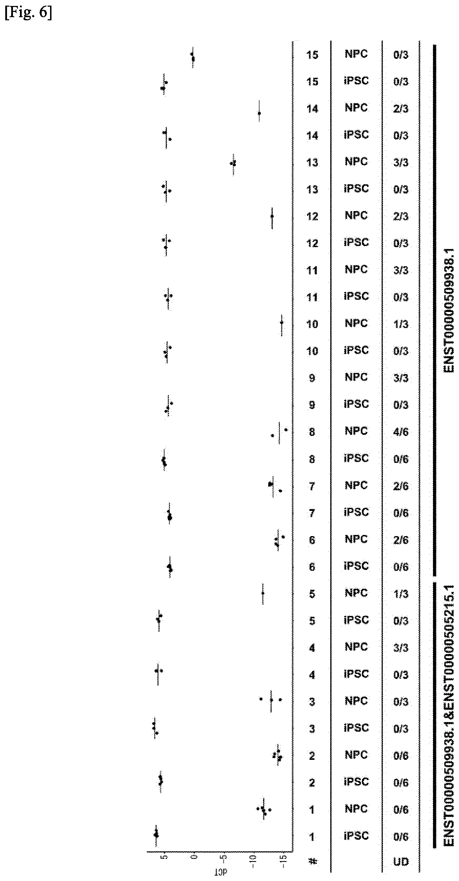

Further, using each combination of primers in Table 1 above that target lncRNA expressed from MIR302CHG, quantitative RT-PCR analysis was performed on human iPSCs and NPCs, respectively. Fig. 6 depicts the results. With any combination of primers, iPSCs exhibited significantly higher comparative Ct (dCt) values compared to NPCs.

### (Example 2)

### Cell enrichment and detection of pluripotent stem cells by quantitative PCR

iPSCs accounting for 10⁻⁴% of the total cell count were mixed with a cell population of nephron progenitor cells (NPCs) to yield 10⁻⁴% iPSCs.

10⁻⁴% iPSCs were subjected to magnetic bead-based cell enrichment using TRA-1-60 surface antigen as an indicator, thereby collecting TRA-1-60 positive fractions (PF) and negative fractions (NF).

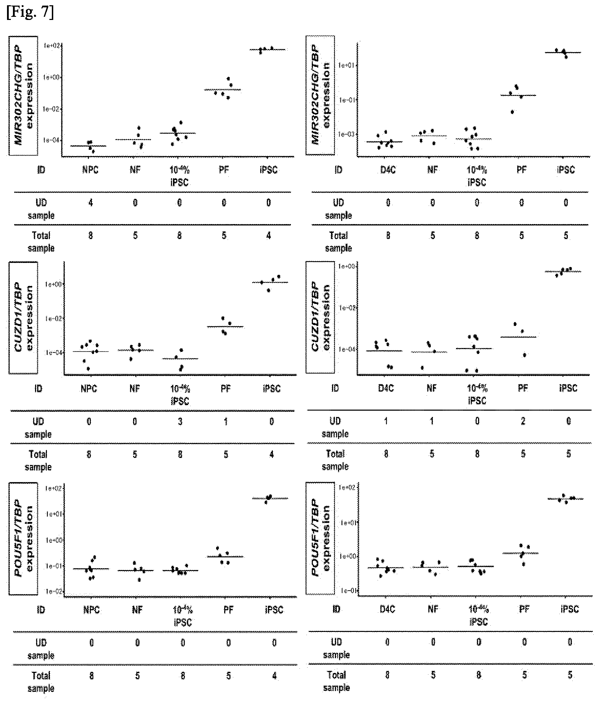

MIR302CHG, CUZD1, and POU5F1 were analyzed for amplifiability by quantitative RT-PCR in each compartment of NPC, iPSC, 10⁻⁴% iPSC, TRA-1-60 PF, and TRA-1-60 NF. For MIR302CHG, primers of the combination of SEQ ID NOS: 6 and 7 (corresponding to #2 in Table 1 above) were used. Fig. 7 depicts the results.

With the combination of magnetic bead-based enrichment and quantitative RT-PCR that measures the level of RNA expressed from MIR302CHG, it is possible to detect the contamination with 10⁻⁴% iPSCs in NPCs or D4C, that is, one or more iPSCs in every 10⁶ cells. On the other hand, in the cases of CUZD1 and POU5F1, it was difficult to detect the contamination with one or more iPSCs in 10⁶ NPCs or D4Cs, or both of them, even with a similar technique.

### (Example 3)

### Detection of pluripotent stem cells by 2-step ddPCR

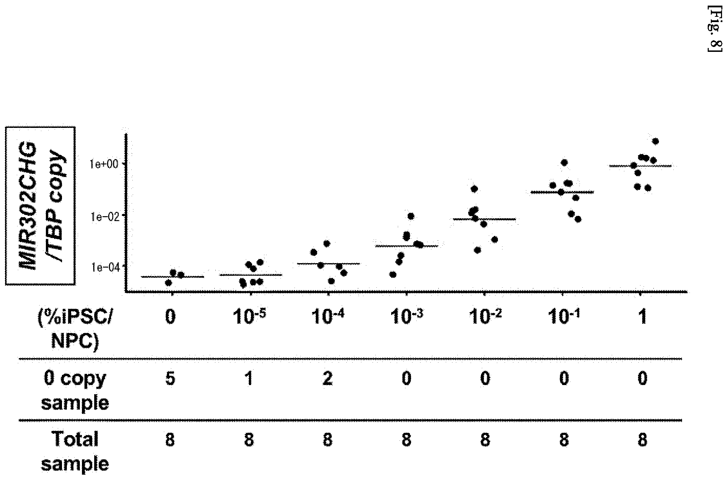

MIR302CHG was subjected to analysis by quantitative RT-PCR using the mixtures of cDNAs derived from human iPSCs and NPCs, which had been prepared by mixing them at 100% to 10⁻⁵% in increments of 10⁻¹. Note that primers of the combination of SEQ ID NOS: 34 and 35 targeting variant 938 expressed from MIR302CHG and the probe of SEQ ID NO: 36 were used. Fig. 8 depicts the results.

It was demonstrated that contamination with at least 10⁻⁴% iPSCs resulted in the higher level of RNA expressed from MIR302CHG than that of NPCs, and in particular, contamination with at least 10⁻²% iPSCs resulted in clearly higher expression level. Therefore, it is concluded that through the use of 2-step ddPCR that measures the level of RNA expressed from MIR302CHG, contamination of NPCs with 10⁻⁴% to 10⁻²% iPSCs, that is, contamination with one or more iPSCs per 10⁶ to 10⁴ cells can be detected.

### (Example 4)

### Expression of MIR302CHG in human iPSC-derived pancreatic progenitor cells and human iPSC-derived hepatocytes

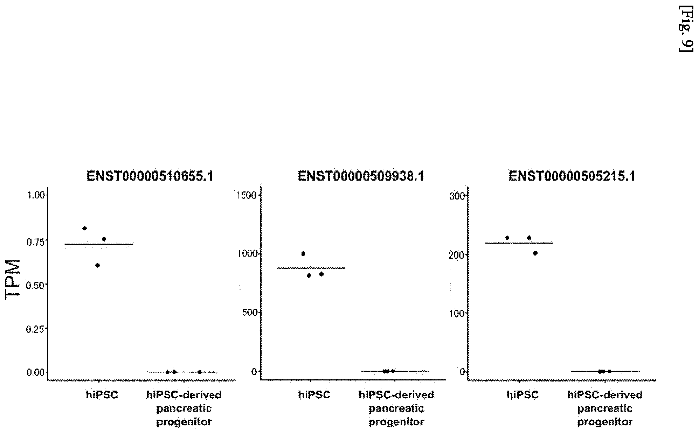

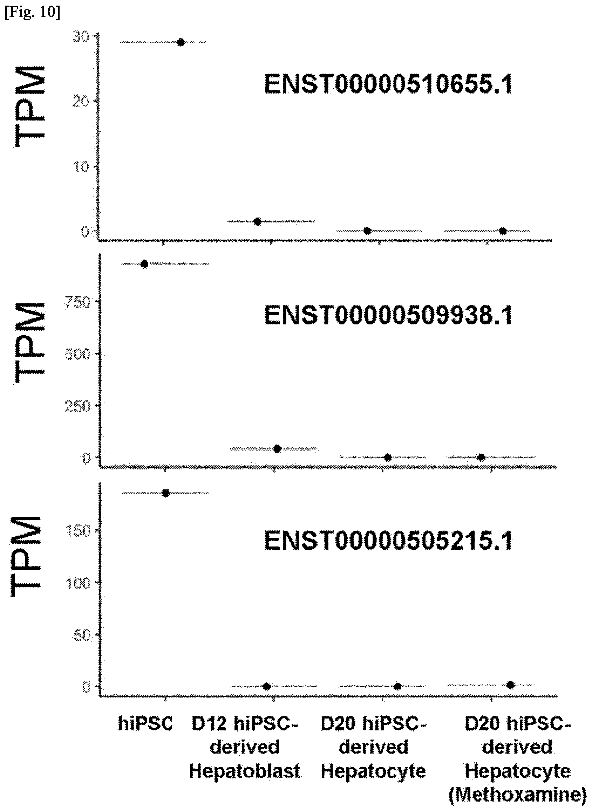

The expression of MIR302CHG in human iPSC-derived pancreatic progenitor cells and liver organoids was analyzed based on publicly available RNA-seq datasets. Fig. 9 depicts the TPM value of each transcript variant of MIR302CHG as analyzed using each publicly available RNA-seq dataset of human iPSCs or human iPSC-derived pancreatic progenitor cells reported in Kimura et al., (2020), Cell Chem. Biol. 27, 1561-1572. Fig. 10 depicts the same as analyzed using each publicly available RNA-seq dataset of human iPSCs or human iPSC-derived hepatocytes reported in Kotaka, M. et al., 2017, Sci. Rep. 7, 1-13.

In both cases, as in the comparison with NPCs, high expression of MIR302CHG was observed in iPSCs, but the expression almost disappeared due to differentiation. Therefore, undifferentiated cells can also be detected using MIR302CHG even during differentiation into the pancreatic lineage and the hepatic lineage.

### (Example 5)

### Expression of MIR302CHG in human iPSC-derived neural progenitor cells and neurons

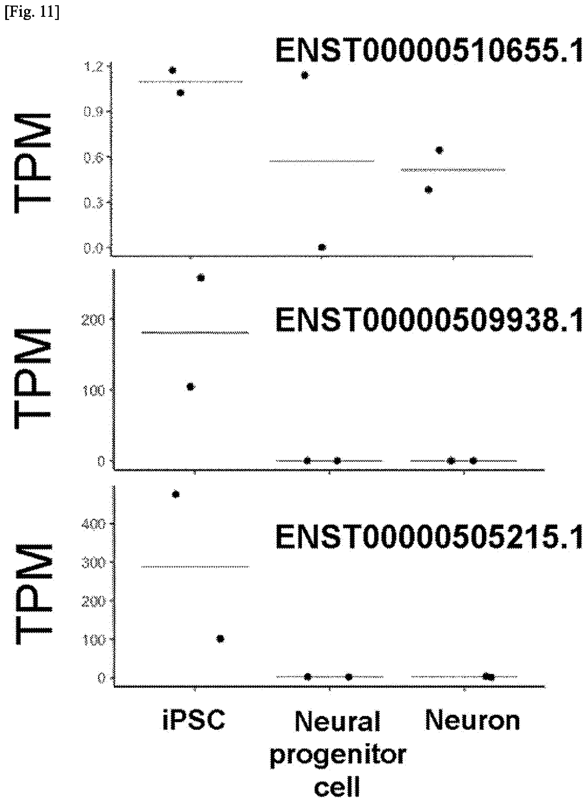

The expression of MIR302CHG in human iPSC-derived neural progenitor cells and neurons was analyzed based on publicly available RNA-seq datasets. Fig. 11 depicts the TPM value of each transcript variant of MIR302CHG as analyzed using the published RNA-seq dataset of human iPSCs or human iPSC-derived neural progenitor cells and neurons reported in Chen et al., 2013, PLoS One. 2013;8: e75682. doi: 10. 1371/journal.pone.0075682.

The high expression of MIR302CHG was observed in iPSCs, but the expression almost disappeared due to differentiation. Therefore, undifferentiated cells can be detected using MIR302CHG even during differentiation into the neuronal lineage.

### (Example 6)

### Expression of MIR302CHG in human iPSC-derived cardiomyocytes

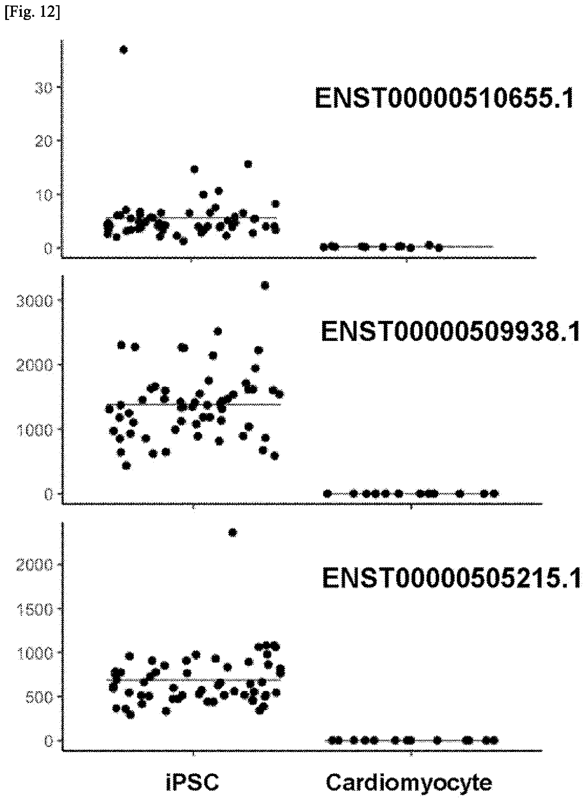

The expression of MIR302CHG in human iPSC-derived cardiomyocytes was analyzed based on publicly available RNA-seq datasets. Fig. 12 depicts the TPM value of each transcript variant of MIR302CHG as analyzed using the publicly available RNA-seq datasets of human iPSCs or human iPSC-derived cardiomyocytes reported in Banovich et al., 2018., Genome Res. 2018; 28: 122-131. doi:10.1101/gr.224436.117.

The high expression of MIR302CHG was observed in iPSCs, but the expression almost disappeared due to differentiation. Therefore, undifferentiated cells can be detected using MIR302CHG even during differentiation into the myocardial lineage.

### (Example 7)

### Expression of MIR302CHG in human ES cell (ESC)-derived pneumocytes

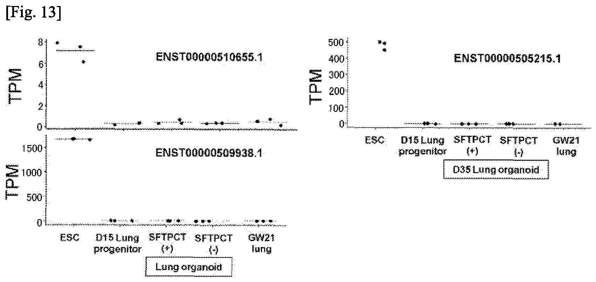

The expression of MIR302CHG in human ES cell-derived lung progenitor cells or lung organoids was analyzed based on publicly available RNA-seq datasets. Fig. 13 depicts the TPM value of each transcript variant of MIR302CHG as analyzed using the publicly available RNA-seq datasets of human ESCs, human ESC-derived lung progenitor cells, or lung organoids reported in Jacob A et al., Cell Stem Cell. 2017; 21: 472-488.e10. doi:10.1016/j.stem.2017.08.014.

The high expression of MIR302CHG was observed in iPSCs, but the expression almost disappeared due to differentiation. Therefore, undifferentiated cells can be detected using MIR302CHG even during differentiation into pneumocytes.

### (Example 8)

### Expression of MIR302CHG in human iPSCs, IVF-ESCs, or nt-ESC-derived vascular endothelial cells

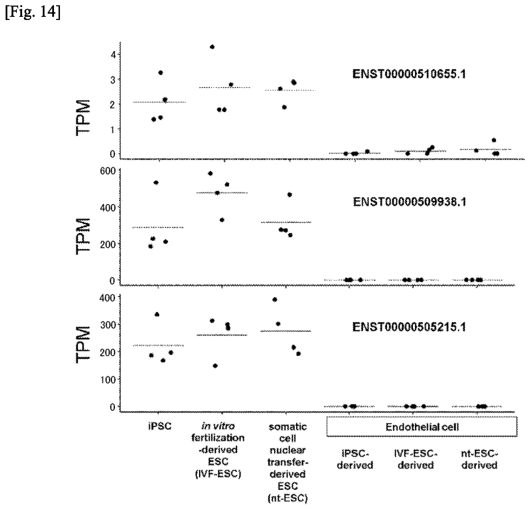

The expression of MIR302CHG in human iPSCs, IVF-ESCs, or nt-ESCs and vascular endothelial cells derived therefrom was analyzed based on publicly available RNA-seq datasets. Fig. 14 depicts the TPM value of each transcript variant of MIR302CHG as analyzed using the publicly available RNA-seq datasets of human iPSCs, IVF-ESCs, nt-ESCs or human iPSCs, IVF-ESCs, and nt-ESC-derived vascular endothelial cells reported in Zhao et. al., Proc Natl Acad Sci U.S.A. 2017;114: E11111-E11120.

The high expression of MIR302CHG was observed in iPSCs, but the expression almost disappeared due to differentiation. Therefore, undifferentiated cells can be detected using MIR302CHG even during differentiation into vascular endothelial cells.

### (Example 9)

### Expression of MIR302CHG in human ESC, anterior primitive streak, definitive endoderm, anterior foregut, posterior foregut, and midgut/hindgut

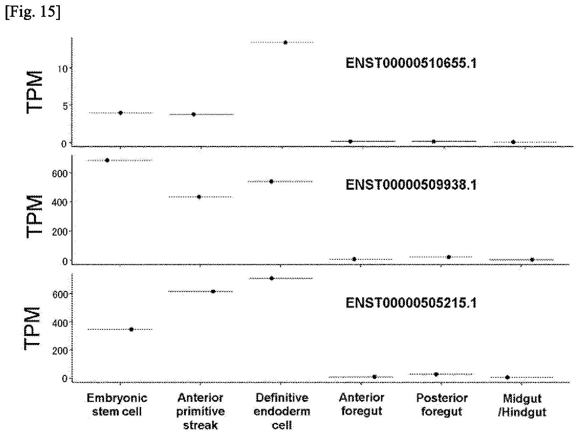

The expression of MIR302CHG in human ESCs, and anterior primitive streak, definitive endoderm, anterior foregut, posterior foregut and midgut/hindgut induced from the human ESCs was analyzed. As a result, as depicted in Fig. 15, it was found that MIR302CHG was expressed in ESCs, anterior primitive streak, and definitive endoderm, but the expression levels decreased in anterior foregut, posterior foregut, and midgut/hindgut. Therefore, by measuring the amount of MIR302CHG, it is possible to detect not only undifferentiated pluripotent stem cells but also cells in the early stages of differentiation such as definitive endoderm and anterior primitive streak, and to distinguish such cells from cells in the later stages of differentiation, such as cells that arise at the foregut, the midgut, and the hindgut stages or later.

### (Example 10)

### Expression of MIR302CHG in renal interstitial progenitor cells and ureteroblasts

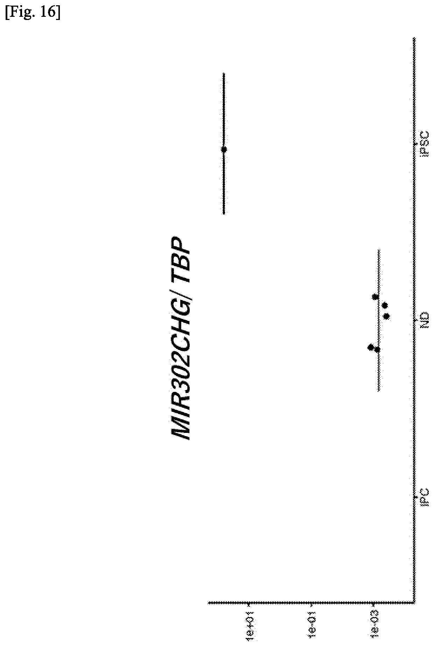

According to the description concerning "renal interstitial progenitor cells" and "ureteroblasts" above, renal interstitial progenitor cells (IPCs) and ureteroblasts (ND) were obtained from human iPSCs, respectively. MIR302CHG was subjected to analysis by quantitative RT-PCR in the IPC and ND, and human iPSC compartments (N=4 for IPC, N=5 for ND). The measured values were normalized with the housekeeping gene TBP (MIR302CHG/TBP). Fig. 16 depicts the results.

In IPCs, the expression level of MIR302CHG was so low such that it was not detected. Even in ND, the expression level of MIR302CHG was as extremely low as approximately 1/10,000 compared to that in iPSCs. In other words, the expression of MIR302CHG, the high-level expression of which was observed in iPSCs, was hardly observed even after differentiation into renal interstitial progenitor cells or ureteroblasts. Therefore, undifferentiated cells can be detected by MIR302CHG even during differentiation into renal interstitial progenitor cells and differentiation into ureteroblasts.

### Industrial Applicability

According to the present invention, undifferentiated pluripotent stem cells remaining in a cell population containing nephron progenitor cells obtained by differentiation induction from pluripotent stem cells can be detected. This makes it possible to assess the safety of products for regenerative medicine and others (for cell therapy, transplant organ reconstruction, etc.) containing nephron progenitor cells derived from human iPS cells.

Furthermore, it is expected that residual undifferentiated pluripotent stem cells are detected in other differentiated cell culture systems other than nephron progenitor cells. Therefore, the present invention is also applicable to products for regenerative medicine and others produced using other differentiated cells other than human iPS cell-derived nephron progenitor cells, such as pancreatic progenitor cells, hepatic progenitor cells, ureteroblasts, interstitial progenitor cells (e.g., renal interstitial progenitor cells), vascular endothelial cells, chondrocytes, bile duct progenitor cells, erythropoietin-producing cells, neurons, cardiomyocytes, and pneumocytes.

## Claims

1. A method for detecting an undifferentiated pluripotent stem cell(s), comprising
(A) a step of measuring the level of RNA expressed from MIR302CHG in a cell population that may contain an undifferentiated pluripotent stem cell(s).

2. The method according to claim 1, wherein the pluripotent stem cell(s) is an induced pluripotent stem (iPS) cell(s).

3. The method according to claim 1, wherein the RNA expressed from MIR302CHG is a splice variant ENST00000509938.1 and/or a splice variant ENST00000505215.1.

4. The method according to claim 3, wherein the RNA expressed from MIR302CHG is the splice variant ENST00000509938.1.

5. The method according to any one of claims 1 to 4, wherein the RNA expressed from MIR302CHG is
(a) a nucleotide sequence of SEQ ID NO: 2 or SEQ ID NO: 3, or
(b) a nucleotide sequence having an identity of 90% or more with the nucleotide sequence of SEQ ID NO: 2 or SEQ ID NO: 3.

6. The method according to any one of claims 1 to 4, wherein the step (A) comprises a step of determining amplifiability by quantitative PCR or a step of determining amplifiability by 2-step ddPCR (Droplet digital PCR).

7. The method according to claim 6, wherein the step of determining amplifiability by quantitative PCR comprises using an oligonucleotide primer set(s) having one or more pairs of sequences selected from the group consisting of SEQ ID NO: 4 and SEQ ID NO: 5, SEQ ID NO: 6 and SEQ ID NO: 7, SEQ ID NO: 8 and SEQ ID NO: 9, SEQ ID NO: 10 and SEQ ID NO: 11, SEQ ID NO: 12 and SEQ ID NO: 13, SEQ ID NO: 14 and SEQ ID NO: 15, SEQ ID NO: 16 and SEQ ID NO: 17, SEQ ID NO: 18 and SEQ ID NO: 19, SEQ ID NO: 20 and SEQ ID NO: 21, SEQ ID NO: 22 and SEQ ID NO: 23, SEQ ID NO: 24 and SEQ ID NO: 25, SEQ ID NO: 26 and SEQ ID NO: 27, SEQ ID NO: 28 and SEQ ID NO: 29, SEQ ID NO: 30 and SEQ ID NO: 31, and SEQ ID NO: 32 and SEQ ID NO: 33.

8. The method according to claim 6, wherein the step of determining amplifiability by 2-step ddPCR comprises using an oligonucleotide probe comprising the nucleotide sequence of SEQ ID NO: 36 and an oligonucleotide primer set having the sequences of SEQ ID NO: 34 and SEQ ID NO: 35.

9. The method according to any one of claims 1 to 4, comprising a step of determining that an undifferentiated pluripotent stem cell(s) contained in a cell population is detected when the level of RNA in the cell population measured in the step (A) is 0.000001 or more with respect to the level of RNA in the cell population consisting of a pluripotent stem cell(s) being set as 1.

10. The method according to any one of claims 1 to 4, comprising a step of enriching a fraction containing the undifferentiated pluripotent stem cell(s) by cell sorting using an undifferentiated pluripotent stem cell-specific antigen(s) prior to the step (A).

11. The method according to claim 10, wherein the undifferentiated pluripotent stem cell-specific antigen(s) is TRA-1-60.

12. The method according to any one of claims 1 to 4, wherein the cell population comprises a nephron progenitor cell(s), a pancreatic progenitor cell(s), a hepatic progenitor cell(s), an ureteroblast(s), an interstitial progenitor cell(s), a vascular endothelial cell(s), a chondrocyte(s), a bile duct progenitor cell(s), an erythropoietin-producing cell(s), a neuron(s), a cardiomyocyte(s), and/or a pneumocyte(s) obtained by differentiation induction from a pluripotent stem cell(s), or a cell(s) obtained at a stage during differentiation from a pluripotent stem cell(s) into any of these cells.

13. The method according to any one of claims 1 to 4, wherein the cell population comprises a nephron progenitor cell(s) obtained by differentiation induction from a pluripotent stem cell(s) or a cell(s) obtained at a stage during differentiation from a pluripotent stem cell(s) into a nephron progenitor cell(s).

14. A reagent kit for detecting an undifferentiated pluripotent stem cell(s),
comprising (i) oligonucleotide primers for measuring the level of RNA expressed from MIR302CHG or (ii) a combination of the oligonucleotide primers and an oligonucleotide probe.

15. The reagent kit according to claim 14, wherein (i) the oligonucleotide primers are an oligonucleotide primer set(s) having one or more pairs of sequences selected from the group consisting of SEQ ID NO: 4 and SEQ ID NO: 5, SEQ ID NO: 6 and SEQ ID NO: 7, SEQ ID NO: 8 and SEQ ID NO: 9, SEQ ID NO: 10 and SEQ ID NO: 11, SEQ ID NO: 12 and SEQ ID NO: 13, SEQ ID NO: 14 and SEQ ID NO: 15, SEQ ID NO: 16 and SEQ ID NO: 17, SEQ ID NO: 18 and SEQ ID NO: 19, SEQ ID NO: 20 and SEQ ID NO: 21, SEQ ID NO: 22 and SEQ ID NO: 23, SEQ ID NO: 24 and SEQ ID NO: 25, SEQ ID NO: 26 and SEQ ID NO: 27, SEQ ID NO: 28 and SEQ ID NO: 29, SEQ ID NO: 30 and SEQ ID NO: 31, and SEQ ID NO: 32 and SEQ ID NO: 33, or (ii) the combination of primers and a probe is the combination of the oligonucleotide primer set having the sequences of SEQ ID NO: 34 and SEQ ID NO: 35, and the oligonucleotide probe comprising the nucleotide sequence of SEQ ID NO: 36.
